# FASCICULE DE BREVET EUROPEEN

(11) **EP 3 013 792 B1**
(45) Date de publication et mention de la délivrance du brevet: **17.01.2018**
(21) Numéro de dépôt: 14736905.2
(22) Date de dépôt: 16.06.2014
(51) Int. Cl.: C07C 237/06, C07C 237/22, C11D 1/52, C11D 3/32, A61K 8/42, A61Q 19/00, B01J 13/00, C09D 5/02, C09D 7/00, D06M 13/418

(54) **AMIDE GRAS A BASE DE CAPROLACTAME COMME ADDITIF GELATEUR.**
CAPROLACTAM-BASIERTES FETTSÄUREAMID ALS GELIERUNGSADDITIV
CAPROLACTAM-BASED FATTY AMIDE AS GELLING ADDITIVE

(30) Priorité: 28.06.2013 FR 1356319
(43) Date de publication de la demande: 04.05.2016
(73) Titulaire: Arkema France, 92700 Colombes (FR)
(72) Inventeur: BERNARD, Michael Y., F-95880 Enghien-les-Bains (FR)
(74) Mandataire: Killis, Andréas
(86) Numéro de dépôt international: PCT/FR2014/051472
(87) Numéro de publication internationale: WO 2014/207344

(56) Documents cités:
- FR-A1- 2 249 871
- M. SUZUKI, ET AL.: "L-Lysine-based low-molecular-weight gelators", CHEMICAL SOCIETY REVIEWS, vol. 38, no. 4, 9 février 2009 (2009-02-09), pages 967-975, XP055116388, Royal Society of Chemistry, Cambridge, GB ISSN: 0306-0012, DOI: 10.1039/b816192e cité dans la demande
- T. HOLAS, ET AL.: "Synthesis and transdermal permeation-enhancing activity of ketone, amide, and alkane analogues of Transkarbam 12", BIOORGANIC & MEDICINAL CHEMISTRY, vol. 14, no. 9, 11 janvier 2006 (2006-01-11), pages 2896-2903, XP027992551, Elsevier Science Publishers, Oxford, GB ISSN: 0968-0896, DOI: 10.1016/j.bmc.2005.12.006 [extrait le 2006-05-01]

## Description

La présente invention concerne un additif amide gras à terminaison amine ou amine salifié et utilisé en tant que gélateur pour milieux organiques ou aqueux, en particulier comme hydrogélateur pour milieux aqueux comme des revêtements ou des colles ou adhésifs ou traitement de fibres ou de textiles ou pour détergents, décapants, dépolluants, floculants.

Divers systèmes pouvant gélifier dans l'eau ou dans un solvant organique avec additifs respectivement hydrogélateurs et organogélateurs sont déjà connus et utilisés dans diverses applications.

FR 2976948 décrit une association ternaire d'un acide de bore, comme l'acide borique, avec un N-alkyl aldonamide en particulier N-dodécyl-D-glucoanamide et d'un sel monovalent tel que NaCl pour l'obtention de gels en milieu aqueux (salin) pour des applications diverses et en particulier dans la détergence.

Parmi les hydrogélateurs connus, figurent ceux à base de dérivés de lysine comme décrits par M.Suzuki et al dans Chem. Soc. Rev., 2009, 38, 967-975, où sont décrits également des dérivés utilisés comme organogélateurs. Parmi ces dérivés sont décrits des diurées-esters, diamide esters ou amide-urée-esters à partir de lysine.

Une revue d'hydrogélateurs a été réalisée par L.A.Estroff et al dans Chemical Reviews, 2004, 104, 3, 1201-1217 avec un inventaire des méthodes de caractérisation et des structures connues. Sont distingués entre autres les dérivés amphiphiles conventionnels ayant une tête hydrophile et une ou deux chaînes hydrophobes, les bola-amphiphiles ayant deux têtes hydrophiles liées par une chaîne hydrophobe, les tensioactifs à double tête ionique séparée par un espaceur rigide avec deux chaînes terminales flexibles, systèmes dérivés des sucres. Il est reconnu qu'il n'y a pas de règle applicable généralement qui permet de trouver le bon compromis entre hydrophilie et hydrophobie d'une molécule et en conséquence il n'y a pas non plus de règle générale entre l'aptitude à former un gel en milieu aqueux et la tendance à éviter la précipitation des fibres.

L'inconvénient de ces hydrogélateurs ou organogélateurs est le fait qu'ils sont à base d'aminoacides qui peuvent subir des réactions secondaires et en particulier des allongements de chaîne incontrôlés suivant les conditions de préparation desdits gélateurs et ainsi affecter leur structure fine et par conséquent leur performance de gélateur. D'autre part, aucun des documents cités ne décrit ni n'enseigne comment obtenir des additifs amides modifiés par le caprolactame et avec des performances améliorées, objet de la présente invention pour remédier aux inconvénients de l'état de la technique.

En effet, la présente invention cherche à mettre au point des nouveaux additifs amides modifiés par une structure caprolactame (équivalente à un amino acide en C₆) sans avoir recours à des acides aminés ou à des acides et à des amines réagissant par polycondensation et en plusieurs étapes nécessitant des chlorures d'acyle.

Ces nouveaux amides doivent permettre l'utilisation d'un procédé de préparation simple et pratique à mettre en oeuvre, avec l'ouverture contrôlée du cycle du caprolactame, afin d'éviter des réactions secondaires difficiles à éviter avec un aminoacide équivalent ou par condensation acide-amine et sans besoin d'étapes de séparation et/ou de purification du produit final. Lesdits nouveaux amides doivent présenter des performances rhéologiques de gélateur satisfaisantes en milieu organique ou aqueux et en particulier en milieu aqueux, sans affecter les performances propres des liants, en particulier aqueux, auxquels ils peuvent être associés.

Le premier objet de l'invention concerne un additif gélateur à base d'amide gras qui est le produit de réaction d'addition (sans élimination de sous-produits) d'une amine grasse avec du caprolactame dans un rapport spécifique contrôlé avec une composition spécifique.

Donc, le premier objet de l'invention est un additif gélateur à base d'amide gras qui est le produit de réaction d'addition d'une amine grasse R₁NH₂ sur du caprolactame avec une fonction amine terminale sous forme salifiée de sel d'ammonium, par neutralisation de ladite fonction amine par un agent neutralisant et qu'il (ledit additif) est constitué de ou comprend le mélange de 3 composés amides différents résultant de ladite réaction (condensation) et caractérisés par le nombre n d'unités caprolactame incorporées qui est respectivement de 1, de 2 et de 3, avec un nombre n moyen d'unités (moyenne par molécule) allant de 0,8 à 3, de préférence de 0,9 à 2,75 et plus préférentiellement allant de 0,9 à 2,5, et en ce que ledit agent neutralisant est sélectionné parmi les acides organiques ou minéraux. Plus particulièrement, lesdits 3 composés amides peuvent être définis selon la formule (I) et correspondent respectivement à n = 1, n = 2 et n = 3

R₁-NH-[-C(=O)(CH₂)₅NH]ₙ₋₁C(=O)(CH₂)₅-Y (I)

avec Y étant -NH₂ ou -NH₃⁺X⁻, si l'amine est sous forme salifiée et avec X⁻ étant un contre anion organique ou minéral lié à l'agent neutralisant acide utilisé X-H.

De préférence, ladite amine grasse R₁NH₂ comprend un nombre d'atomes de carbone allant de 10 à 24, ce qui signifie que R₁ est un alkyl en C₁₀ à C₂₄ et de préférence R₁ est linéaire.

Comme exemples convenables de R₁NH₂, on peut citer les monoamines grasses telles que la décyl amine, undécyl amine, dodécyl amine (ou lauryl amine), la tridécyl amine, la tetradécyl amine, la pentadécyl amine, l'hexadécyl amine, l'heptadécyl amine, l'octadécyl amine (ou stéaryl amine), l'eicosanamine, doeicosanamine ou tetraeicosanamine ou leurs isomères et leurs mélanges.

Comme exemples convenables d'acide minéral, on peut citer entre autres, l'acide chlorhydrique, l'acide sulfurique, l'acide phosphorique, l'acide borique, l'acide nitrique.

Comme exemples convenables d'acides organiques, on peut citer les acides carboxyliques, les acides sulfoniques, les acides phosphoniques et phosphiniques.

Ledit additif de l'invention peut être utilisé en particulier sous forme de gel dans l'eau ou sous forme de gel dans un solvant organique de préférence à un taux en poids inférieur à 5% et plus préférentiellement à un taux ne dépassant pas 1% en poids, ce pourcentage étant défini par rapport au poids de l'eau + additif et encore plus préférentiellement ledit additif est utilisé en tant qu'hydrogélateur sous forme de gel dans l'eau ou en d'autres termes sous forme d'hydrogel.

Ledit additif peut être sous la forme d'une poudre micronisée, de préférence ayant une taille moyenne en volume inférieure à 50 µ, de préférence inférieure à 25 µ. Cette granulométrie peut être déterminée directement sur la poudre sèche par diffraction laser. Cette technique est basée sur le principe que des particules passant à travers un faisceau laser diffractent la lumière selon un angle différent en fonction de leur taille : les particules de petites tailles diffractent aux grands angles, alors que les particules de tailles plus importantes diffractent aux petits angles.

Ledit solvant organique est de préférence un solvant organique polaire ou un solvant organique qui est un mélange homogène sans démixtion et qui comprend un solvant organique polaire, tel qu'un alcool en C₁ à C₄, diméthyl sulfoxyde (DMSO) ou diméthyl formamide (DMF), N-methyl pyrrolidone (NMP), N-ethyl pyrrolidone (NEP) ou un plastifiant organique polaire.

Le deuxième objet de l'invention concerne un procédé de préparation de l'additif gélateur selon l'invention, lequel procédé comprend une étape de réaction d'addition entre une amine grasse R₁NH₂ et le caprolactame avec un rapport molaire dudit caprolactame par rapport à ladite amine grasse, allant de 0,8 à 3, de préférence de 0,9 à 2,75 et plus préférentiellement de 0,9 à 2,5, ledit procédé ne comprenant aucune étape de séparation ni de purification. Cette réaction est une réaction d'addition en masse à l'état fondu, qui peut avoir lieu à une température allant de 200°C à 300°C° et sous atmosphère inerte. Après refroidissement, le produit obtenu est micronisé par broyage mécanique ou jet d'air. Un tamisage peut permettre d'obtenir une granulométrie fine et contrôlée avec une taille moyenne en volume inférieure à 50 µ, de préférence inférieure à 25 µ, mesurée par diffraction laser, comme par exemple sur le Mastersizer® S de Malvern. Un catalyseur peut être utilisé pour cette réaction, comme par exemple un acide de Lewis ou une base de Lewis.

Un autre objet de l'invention concerne une composition aqueuse ou composition en milieu solvant organique, de préférence composition aqueuse, laquelle comprend au moins un additif tel que défini ci-haut ou obtenu par le procédé tel que défini ci-dessus selon l'invention, ledit additif étant utilisé en tant que gélateur et en particulier pour composition aqueuse en tant qu'hydrogélateur et plus particulièrement en tant qu'additif de rhéologie. De préférence, ladite composition est aqueuse et a un pH acide de préférence inférieur à 5, plus préférentiellement inférieur à 4.

Ce pH est également valable pour l'additif présent sous forme de gel dans l'eau (hydrogel).

Ce pH peut être ajusté par addition dudit acide organique ou minéral en excès.

Selon une option plus spécifique, ladite composition est une composition aqueuse de liant organique et en particulier une composition de revêtements, plus particulièrement en ce qui concerne une composition de revêtements parmi vernis, peintures, encres ou une composition de colles ou d'adhésifs ou de cosmétique ou une composition aqueuse de traitement de fibres ou de textiles et ledit additif hydrogélateur est un additif de rhéologie. Dans cette option de composition, ladite composition aqueuse est une composition de liant organique et comprend donc en plus dudit additif au moins un liant organique lié à l'application visée. Selon une autre option de composition aqueuse, celle-ci peut ne pas comprendre de liant organique et être une composition dudit additif dans l'eau sous forme d'hydrogel ou elle peut être une composition de tensioactif, en particulier de détergent ou d'agent décapant ou d'agent dépolluant ou d'agent floculant.

Dans le cas où ledit additif est utilisé comme organogélateur en milieu solvant organique (ou milieu organique), de préférence polaire, ladite composition en milieu organique qui en résulte peut être une composition dudit additif dans ledit solvant sous forme d'organogel ou elle peut être une composition de revêtements, en particulier parmi peintures, vernis, encres ou gel coats ou de colles ou d'adhésifs ou composition de mastics ou d'agent d'étanchéité ou d'agent décapant ou une composition de moulage.

Sont plus particulièrement préférées les compositions aqueuses comprenant ledit additif en tant qu'hydrogélateur.

L'invention concerne également l'utilisation d'un additif amide gras tel que défini ci-haut selon l'invention en tant qu'additif gélateur pour compositions en milieu solvant organique, en tant qu'organogélateur ou pour compositions aqueuses en tant qu'hydrogélateur. Plus particulièrement, ledit additif est utilisé en tant qu'hydrogélateur dans l'eau pour l'obtention d'un hydrogel ou dans une composition aqueuse de détergent, d'agent dépolluant, d'agent décapant, d'agent floculant ou dans une composition aqueuse de revêtements ou de colles ou d'adhésifs ou de cosmétique ou une composition aqueuse de traitement de fibres ou de textiles. La composition aqueuse de revêtements est particulièrement préférée où ledit additif hydrogélateur est utilisé comme additif de rhéologie.

Ledit additif peut aussi être utilisé, en particulier sous forme non salifiée, en tant qu'organogélateur dans un solvant organique, de préférence polaire, pour l'obtention d'un organogel ou dans une composition en milieu solvant organique, en particulier dans une composition de revêtements parmi peintures, vernis, encres ou gel coats ou dans une composition de colles ou d'adhésifs ou dans une composition cosmétique ou une composition de moulage ou une composition de mastics ou d'agent d'étanchéité ou d'agent décapant.

La force de gel ou l'intensité de gélification dépend et peut être adaptée en fonction de l'application visée et du milieu utilisé.

L'invention couvre également un gel parmi organogel ou hydrogel, en particulier hydrogel, qui est le produit résultant de l'utilisation dudit additif. Il s'agit d'organogel dans un solvant ou milieu organique ou d'hydrogel dans l'eau ou en milieu aqueux.

Finalement, fait également partie de l'invention le produit final résultant de l'utilisation en milieu aqueux en tant qu'hydrogélateur et plus particulièrement comme additif de rhéologie, d'au moins un additif tel que défini ci-haut ou obtenu par un procédé tel que défini ci-haut selon l'invention et en particulier ledit produit final étant sélectionné parmi revêtement ou colle ou adhésif ou cosmétique ou détergent ou agent décapant ou agent dépolluant ou fibre traitée ou textile traité.

Les exemples suivants sont présentés à titre d'illustration de l'invention et de ses performances et de ce fait ne limitent en rien sa couverture.

### Partie expérimentale

### I - Matières premières utilisées

**Tableau 1 : Matières premières utilisées**

| **Produit** | **Fonction** | **Référence commerciale** | **Fournisseur** |
|---|---|---|---|
| Octadecylamine | Réactif | Octadecylamine 97% | ALDRICH |
| Caprolactame | Réactif | ε-Caprolactam | ALDRICH |
| Carboxylate de zinc | Catalyseur | Borchikat® 22 | OMG BORCHERS SAS |
| Acide chlorydrique | Acide | Hydrochloric acid ACS reagent, 37% | ALDRICH |

### II - Exemples de préparation d'amides hydrogélateurs à base de caprolactame

### Exemple 1 : préparation de l'amide A1 par réaction de 1 mole d'octadécylamine avec 1 mole de caprolactame :

Dans un réacteur de 1-Litre équipé d'un thermomètre, un condensateur et d'un agitateur, on introduit sous un courant d'azote, 113,16 g de Caprolactame (1 mole), 274,84 g d'octadecylamine (1 mole) et 1,95 g de Borchikat® 22.

Le mélange est chauffé à 250°C toujours sous courant d'azote. La réaction est contrôlée par la viscosité. Après 15 heures, la valeur de viscosité devient constante (et > 0,23 P ou > 23 mPa.s, mesuré sur un Brookfield® CAP1000 à 100°C), le mélange réactionnel est refroidi à 150°C puis déchargé dans un moule siliconé. Une fois refroidi à la température ambiante, le produit est micronisé mécaniquement par broyage. Un tamisage pour obtenir une granulométrie fine et contrôlée avec taille moyenne obtenue de 7 µm est effectué.

### Exemple 2 : préparation de l'amide A2 par réaction de 1 mole d'octadécylamine avec 2 moles de caprolactame:

Dans un réacteur de 1-Litre équipé d'un thermomètre, un condensateur et d'un agitateur, on introduit sous un courant d'azote, 226,32 g de caprolactame (soit 2 moles), 274,84 g d'octadécylamine (soit 1 mole), 1,95 g de Borchikat® 22.

Le reste du mode opératoire est réalisé comme décrit ci-haut pour l'exemple 1.

### III - Evaluation des performances de gélification

### 1. Formulation pour l'évaluation des additifs amides comme hydrogélateurs

Dans un erlenmeyer muni d'un barreau aimanté, on introduit 99 grammes d'eau déminéralisée, 1 g d'additif amide hydrogélateur broyé ou micronisé à tester, puis quelques gouttes d'acide chlorhydrique avec un excès correspondant à plus de 4 fois les équivalents d'amine de l'amide hydrogélateur à tester. L'erlenmeyer est ensuite fermé. Puis, le mélange est agité pendant plus de 5 heures à 85°C pour avoir une parfaite dissolution de l'hydrogélateur et un mélange laiteux mais sans précipité. Enfin, le mélange est introduit dans un tube à essai puis laissé au repos à 25°C pendant 24 heures.

Dans ces conditions de préparation, 3 formulations ont été réalisées avec l'amide A1, l'amide A2 comme décrits respectivement dans les exemples de préparation 1 et 2 et aussi l'octadecylamine à titre de référence de comparaison. Ces 3 essais sont résumés dans le tableau 2 ci-dessous.

**Tableau 2 : Formulations**

| **Réf Essai** | **Additif amide hydrogélateur ou additif comparatif de référence** | **% en poids d'additif dans formulation aqueuse** |
|---|---|---|
| 1 | Octadécylamine (comparatif) | 1% |
| 2 | Amide A1 (invention) | 1% |
| 3 | amide A2 (invention) | 1% |

### 2. Evaluation du gel

Les 3 essais de formulations ont été évalués de deux manières : d'abord selon l'aspect des formulations préparées dans les tubes à essais après 24 heures (voir tableau 3) et ensuite selon leur viscosité à différentes vitesses de cisaillement sur un viscosimètre Brookfield® (voir tableau 4).

**Tableau 3 : Aspect**

| **Réf essai** | **Aspect** |
|---|---|
| 1 | Suspension |
| 2 | Gel |
| 3 | Gel |

**Tableau 4 : Viscosité à différentes vitesses**

| | Vitesse mobile (rpm) | Essai 1 | Essai 2 | Essai 3 |
|---|---|---|---|---|
| Viscosité mesurée au Brookfield à 25°C (mPa.s) | 1 | 3200* | 6700 | 3300 |
| | 5 | 900* | 1860 | 1440 |
| | 10 | 530* | 1080 | 870 |
| | 50 | 174* | 390 | 238 |
| | 100 | 116 | 250 | 126 |

| | | | | |
|---|---|---|---|---|
| * Remarque : les résultats sont peu répétables (reproductibles), la formulation étant non homogène | | | | |

Contrairement à l'octadécylamine, les formulations avec les amides A1 ou A2 selon l'invention se présentent sous forme de gel caractéristique d'un hydrogélateur.

Quant aux résultats de viscosité, ils montrent que la formulation contenant l'amide A1 (essai 2) est thixotropique mais également plus visqueuse que l'eau ou la formulation contenant l'octadécylamine.

## Revendications

1. Additif gélateur à base d'amide gras, **caractérisé en ce qu'**il est le produit de réaction d'addition d'une amine grasse R₁NH₂ sur du caprolactame avec une fonction amine terminale sous forme salifiée de sel d'ammonium, par neutralisation de ladite fonction amine par un agent neutralisant et qu'il est constitué de ou comprend le mélange de 3 composés amides différents résultant de ladite réaction et **caractérisés par** le nombre n d'unités caprolactame incorporées qui est respectivement de 1, de 2 et de 3 avec un nombre n moyen d'unités (moyenne par molécule) allant de 0,8 à 3, de préférence de 0,9 à 2,75 et plus préférentiellement allant de 0,9 à 2,5
et **en ce que** ledit agent neutralisant est sélectionné parmi les acides organiques ou minéraux.

2. Additif selon la revendication 1, **caractérisé en ce que** lesdits 3 composés amides sont définis selon la formule (I) suivante et correspondent respectivement à n = 1, n = 2 et n = 3
R₁-NH-[-C(=O)(CH₂)₅NH]ₙ₋₁-C(=O)(CH₂)₅-Y (I)
avec Y étant -NH₂ ou -NH₃⁺X⁻, si l'amine est sous forme salifiée et avec X⁻ étant un contre anion organique ou minéral lié à l'agent neutralisant acide utilisé X-H.

3. Additif selon la revendication 1 ou 2, **caractérisé en ce que** ladite amine grasse comprend un nombre d'atomes de carbone allant de 10 à 24.

4. Additif selon l'une des revendications 1 à 3, **caractérisé en ce que** ledit additif est sous la forme d'une poudre micronisée, de préférence ayant une taille moyenne en volume inférieure à 50 µ, de préférence inférieure à 25 µ.

5. Additif selon l'une des revendications 1 à 4, **caractérisé en ce qu'**il est sous forme de gel dans l'eau ou sous forme de gel dans un solvant organique, de préférence à un taux en poids inférieur à 5%, plus préférentiellement à un taux ne dépassant pas 1% en poids, encore plus préférentiellement ledit additif étant utilisé en tant qu'hydrogélateur sous forme de gel dans l'eau.

6. Procédé de préparation d'un additif tel que défini selon l'une des revendications 1 à 5, **caractérisé en ce qu'**il comprend une étape de réaction d'addition entre une amine grasse R₁NH₂ et le caprolactame avec un rapport molaire dudit caprolactame par rapport à ladite amine grasse allant de 0,8 à 3, de préférence de 0,9 à 2,75 et plus préférentiellement de 0,9 à 2,5, ledit procédé ne comprenant aucune étape de séparation ou de purification.

7. Composition aqueuse ou composition en milieu solvant organique, de préférence composition aqueuse, **caractérisée en ce qu'**elle comprend au moins un additif tel que défini selon l'une des revendications 1 à 5 ou tel qu'obtenu par le procédé tel que défini selon la revendication 6, en tant que gélateur, en particulier en tant qu'hydrogélateur pour composition aqueuse.

8. Composition selon la revendication 7, **caractérisée en ce qu'**elle est une composition aqueuse et qu'elle a un pH acide, de préférence inférieur à 5, plus préférentiellement inférieur à 4.

9. Composition selon la revendication 7 ou 8, **caractérisée en ce qu'**il s'agit d'une composition aqueuse de tensioactif et en particulier de détergent, d'agent décapant, d'agent dépolluant ou d'agent floculant.

10. Composition aqueuse selon la revendication 7 ou 8, **caractérisée en ce qu'**il s'agit d'une composition aqueuse de liant organique, en particulier une composition de revêtements, de colles, d'adhésifs, de cosmétique ou une composition aqueuse de traitement de fibres ou de textiles avec ledit hydrogélateur étant un additif de rhéologie.

11. Composition selon la revendication 10, **caractérisée en ce qu'**il s'agit d'une composition de revêtements parmi les vernis, de peintures ou les encres.

12. Composition selon la revendication 7, **caractérisée en ce qu'**il s'agit d'une composition en milieu solvant organique qui est une composition dudit additif dans ledit solvant sous forme d'organogel ou une composition de revêtements, en particulier parmi peintures, vernis, encres, gel coats ou une composition de colles ou d'adhésifs ou une composition de mastics ou d'agents d'étanchéité ou d'agent décapant ou une composition de moulage.

13. Utilisation d'un amide gras tel que défini dans l'une des revendications 1 à 5, en tant qu'additif gélateur pour compositions en milieu solvant organique en tant qu'organogélateur ou pour compositions aqueuses en tant qu'hydrogélateur.

14. Utilisation selon la revendication 13, **caractérisée en ce que** ledit additif est utilisé en tant qu'hydrogélateur dans l'eau pour l'obtention d'un hydrogel ou dans une composition aqueuse de détergent, d'agent dépolluant, d'agent décapant, d'agent floculant ou dans une composition aqueuse de revêtements, en particulier de peintures, de vernis ou d'encres ou dans une composition de colles ou d'adhésifs ou dans une composition cosmétique ou dans une composition de traitement de fibres ou de textiles.

15. Utilisation selon le revendication 13, **caractérisée en ce que** ledit additif est utilisé en tant qu'organogélateur dans un solvant organique pour l'obtention d'un organogel ou dans une composition en milieu solvant organique, en particulier dans une composition de revêtements parmi peintures, vernis, encres ou gel coats ou dans une composition de colles ou d'adhésifs ou dans une composition cosmétique ou dans une composition de moulage ou dans une composition de mastics ou d'agent d'étanchéité ou d'agent décapant.

16. Gel parmi organogel ou hydrogel, en particulier hydrogel, **caractérisé en ce qu'**il est le produit résultant de l'utilisation telle que définie selon l'une des revendications 13 à 15 d'un additif tel que défini selon l'une des revendications 1 à 5 ou obtenu par un procédé tel que défini selon la revendication 6.

17. Produit final résultant de l'utilisation en milieu aqueux en tant qu'hydrogélateur et plus particulièrement comme additif de rhéologie, d'au moins un additif tel que défini selon l'une des revendications 1 à 5 ou obtenu par un procédé tel que défini selon la revendication 6, en particulier sélectionné parmi revêtement ou colle ou adhésif ou cosmétique ou détergent ou agent décapant ou agent dépolluant ou fibre traitée ou textile traité.

## Patentansprüche

1. Gelierungadditiv auf Fettamid-Basis, **dadurch gekennzeichnet, dass** es das Produkt einer Additionsreaktion eines Fettamins R₁NH₂ an Caprolactam mit einer terminalen Aminfunktion in Salzform als Ammoniumsalz durch Neutralisierung der Aminfunktion durch ein Neutralisierungsmittel ist und dass es aus einer Mischung aus 3 unterschiedlichen Amidverbindungen besteht oder diese umfasst, die aus der Reaktion resultieren und durch die Zahl von n eingearbeiteten Caprolactam-Einheiten gekennzeichnet sind, die 1, 2 bzw. 3 beträgt, wobei eine mittlere Zahl n von Einheiten (Durchschnitt pro Molekül) von 0,8 bis 3, vorzugsweise von 0,9 bis 2,75 und stärker bevorzugt von 0,9 bis 2,5 reicht,
und dass das Neutralisierungsmittel aus organischen oder mineralischen Säuren ausgewählt ist.

2. Additiv nach Anspruch 1, **dadurch gekennzeichnet, dass** die 3 Amidverbindungen durch die folgende Formel (I) definiert sind und n = 1, n = 2 bzw. n = 3 entsprechen
R₁-NH-[-C(=O)(CH₂)₅NH]ₙ₋₁-C(=O)(CH₂)₅-Y (I)
wobei Y -NH₂ oder -NH₃⁺X⁻ ist, wenn das Amin in Salzform vorliegt, und wobei X⁻ ein organisches oder mineralisches Gegenanion ist, das an das verwendete saure Neutralisierungsmittel X-H gebunden ist.

3. Additiv nach Anspruch 1 oder 2, **dadurch gekennzeichnet, dass** das Fettamin eine Anzahl an Kohlenstoffatomen umfasst, die von 10 bis 24 reicht.

4. Additiv nach einem der Ansprüche 1 bis 3, **dadurch gekennzeichnet, dass** das Additiv in Form eines mikronisierten Pulvers vorliegt, das vorzugsweise eine volumenmittlere Größe unter 50 µ, vorzugsweise unter 25 µ aufweist.

5. Additiv nach einem der Ansprüche 1 bis 4, **dadurch gekennzeichnet, dass** es in Form eines Gels in Wasser oder in Form eines Gels in einem organischen Lösemittel vorliegt, vorzugsweise mit einem Gewichtsanteil von weniger als 5 %, stärker bevorzugt mit einem Anteil, der 1 Gew.-% nicht übersteigt, wobei das Additiv noch stärker bevorzugt als Hydrogelbildner in Form eines Gels in Wasser verwendet wird.

6. Verfahren zur Herstellung eines Additivs, wie es in einem der Ansprüche 1 bis 5 definiert ist, **dadurch gekennzeichnet, dass** es einen Schritt einer Additionsreaktion zwischen einem Fettamin R₁NH₂ und dem Caprolactam umfasst, wobei das Molverhältnis des Caprolactams zu dem Fettamin von 0,8 bis 3, vorzugsweise von 0,9 bis 2,75 und stärker bevorzugt von 0,9 bis 2,5 reicht, wobei das Verfahren keinen Schritt der Trennung oder Reinigung umfasst.

7. Wässrige Zusammensetzung oder Zusammensetzung in einem organischen Lösungsmedium, vorzugsweise wässrige Zusammensetzung, **dadurch gekennzeichnet, dass** sie wenigstens ein Additiv umfasst, wie es in einem der Ansprüche 1 bis 5 definiert ist oder wie es durch das Verfahren erhalten wird, wie es in Anspruch 6 definiert ist, als Gelbildner, insbesondere als Hydrogelbildner für eine wässrige Zusammensetzung.

8. Zusammensetzung nach Anspruch 7, **dadurch gekennzeichnet, dass** sie eine wässrige Zusammensetzung ist und dass sie einen sauren pH, vorzugsweise unter 5, stärker bevorzugt unter 4, aufweist.

9. Zusammensetzung nach Anspruch 7 oder 8, **dadurch gekennzeichnet, dass** es sich um eine wässrige Tensid- und insbesondere Detergens-, Beizmittel-, Schadstoffentfernungsmittel- oder Flockungsmittel-Zusammensetzung handelt.

10. Wässrige Zusammensetzung nach Anspruch 7 oder 8, **dadurch gekennzeichnet, dass** es sich um eine wässrige Zusammensetzung eines organischen Bindemittels, insbesondere eine Beschichtungs-, Klebstoff-, Haftmittel-, Kosmetika-Zusammensetzung, oder um eine wässrige Zusammensetzung zur Behandlung von Fasern oder Textilien handelt, wobei der Hydrogelbildner ein Rheologieadditiv ist.

11. Zusammensetzung nach Anspruch 10, **dadurch gekennzeichnet, dass** es sich um eine Beschichtungszusammensetzung, darunter Lacke, Anstriche oder Tinten, handelt.

12. Zusammensetzung nach Anspruch 7, **dadurch gekennzeichnet, dass** es sich um eine Zusammensetzung in einem organischen Lösungsmedium handelt, die eine Zusammensetzung des Additivs in dem Lösemittel in Form eines Organogels oder eine Beschichtungszusammensetzung, darunter insbesondere Anstriche, Lacke, Tinten, Gelbeschichtungen, oder eine Klebstoff- oder Haftmittel-Zusammensetzung oder eine Kitt- oder Dichtmittel- oder Beizmittel-Zusammensetzung oder eine Formzusammensetzung ist.

13. Verwendung eines Fettamids, wie es in einem der Ansprüche 1 bis 5 definiert ist, als Gelierungadditiv für Zusammensetzungen in einem organischen Lösungsmedium als Organogelbildner oder für wässrige Zusammensetzungen als Hydrogelbildner.

14. Verwendung nach Anspruch 13, **dadurch gekennzeichnet, dass** das Additiv als Hydrogelbildner in Wasser zum Erhalt eines Hydrogels oder in einer wässrigen Detergens-, Schadstoffentfernungsmittel-, Beizmittel-, Flockungsmittel-Zusammensetzung oder in einer wässrigen Beschichtungszusammensetzung, insbesondere Anstriche, Lacke oder Tinten, oder in einer Klebstoff- oder Haftmittel-Zusammensetzung oder in einer Kosmetika-Zusammensetzung oder in einer Zusammensetzung zur Behandlung von Fasern oder Textilien verwendet wird.

15. Verwendung nach Anspruch 13, **dadurch gekennzeichnet, dass** das Additiv als Organogelbildner in einem organischen Lösemittel zum Erhalt eines Organogels oder in einer Zusammensetzung in einem organischen Lösungsmedium, insbesondere in einer Beschichtungszusammensetzung, darunter Anstriche, Lacke, Tinten oder Gelbeschichtungen, oder in einer Klebstoff- oder Haftmittel-Zusammensetzung oder in einer Kosmetika-Zusammensetzung oder in einer Formzusammensetzung oder in einer Kitt- oder Dichtmittel- oder Beizmittel-Zusammensetzung verwendet wird.

16. Gel, darunter Organogel oder Hydrogel, insbesondere Hydrogel, **dadurch gekennzeichnet, dass** es das Produkt ist, das aus der in einem der Ansprüche 13 bis 15 definierten Verwendung eines Additivs resultiert, wie es in einem der Ansprüche 1 bis 5 definiert ist, oder das durch ein Verfahren erhalten wird, wie es in Anspruch 6 definiert ist.

17. Endprodukt, resultierend aus der Verwendung wenigstens eines Additivs, wie es in einem der Ansprüche 1 bis 5 definiert ist oder das durch ein Verfahren erhalten wird, wie es in Anspruch 6 definiert ist, in einem wässrigen Medium als Hydrogelbildner und vor allem als Rheologieadditiv, wobei es insbesondere aus einer Beschichtung oder einem Klebstoff oder einem Haftmittel oder einem Kosmetikum oder einem Detergens oder einem Beizmittel oder einem Schadstoffentfernungsmittel oder einer behandelten Faser oder einem behandelten Textil ausgewählt ist.

## Claims

1. Gelating additive based on fatty amide, **characterized in that** it is the product of the addition reaction of a fatty amine R₁NH₂ with caprolactam with an end amine functional group in the salified ammonium salt form, by neutralization of the said amine functional group by a neutralizing agent, and that it consists of or comprises the mixture of 3 different amide compounds resulting from the said reaction and which are **characterized by** the number n of incorporated caprolactam units, which is respectively 1, 2 and 3, with a mean number n of units (mean per molecule) ranging from 0.8 to 3, preferably from 0.9 to 2.75 and more preferably from 0.9 to 2.5, **in that** and the said neutralizing agent is selected from organic or inorganic acids.

2. Additive according to Claim 1, **characterized in that** the said 3 amide compounds are defined according to the following formula (I) and respectively correspond to n = 1, n = 2 and n = 3
R₁-NH-[-C(=O)(CH₂)₅NH]ₙ₋₁-C(=O)(CH₂)₅-Y (I)
with Y being -NH₂ or -NH₃⁺X⁻, if the amine is in the salified form and with X⁻ being an organic or inorganic counteranion related to the acid neutralizing agent used, X-H.

3. Additive according to Claim 1 or 2, **characterized in that** the said fatty amine comprises a number of carbon atoms ranging from 10 to 24.

4. Additive according to one of Claims 1 to 3, **characterized in that** the said additive is in the form of a micronized powder, preferably having a volume-average size of less than 50 µ, preferably of less than 25 µ.

5. Additive according to one of Claims 1 to 4, **characterized in that** it is in the gel form in water or in the gel form in an organic solvent, preferably at a content by weight of less than 5%, more preferably at a content not exceeding 1% by weight, the said additive more preferably still being used as hydrogelator in the gel form in water.

6. Process for the preparation of an additive as defined according to one of Claims 1 to 5, **characterized in that** it comprises a stage of an addition reaction between a fatty amine R₁NH₂ and caprolactam with a molar ratio of the said caprolactam with respect to the said fatty amine ranging from 0.8 to 3, preferably from 0.9 to 2.75 and more preferably from 0.9 to 2.5, the said process not comprising any separation or purification stage.

7. Aqueous composition or composition in an organic solvent medium, preferably an aqueous composition, **characterized in that** it comprises at least one additive as defined according to one of Claims 1 to 5 or as obtained by the process as defined according to Claim 6 as gelator, in particular as hydrogelator for an aqueous composition.

8. Composition according to Claim 7, **characterized in that** it is an aqueous composition and that it has an acidic pH, preferably of less than 5, more preferably of less than 4.

9. Composition according to Claim 7 or 8, **characterized in that** it is an aqueous surfactant and in particular detergent, stripping agent, depolluting agent or flocculating agent composition.

10. Aqueous composition according to Claim 7 or 8, **characterized in that** it is an aqueous organic binder composition, in particular a coating, adhesive or cosmetic composition or an aqueous composition for the treatment of fibres or textiles, with the said hydrogelator being a rheology additive.

11. Composition according to Claim 10, **characterized in that** it is a coating composition from varnishes, paints or inks.

12. Composition according to Claim 7, **characterized in that** it is a composition in an organic solvent medium which is a composition of the said additive in the said solvent in the organogel form or a coating composition, in particular from paints, varnishes, inks or gel coats, or an adhesive composition or a mastic or leaktightness agent or stripping agent composition or a moulding composition.

13. Use of a fatty amide as defined in one of Claims 1 to 5 as gelating additive for compositions in an organic solvent medium, as organogelator, or aqueous compositions, as hydrogelator.

14. Use according to Claim 13, **characterized in that** the said additive is used as hydrogelator in water, in order to obtain a hydrogel, or in an aqueous detergent, depolluting agent, stripping agent or flocculating agent composition or in an aqueous coating composition, in particular an aqueous paint, varnish or ink composition, or in an adhesive composition or in a cosmetic composition or in a composition for the treatment of fibres or textiles.

15. Use according to Claim 13, **characterized in that** the said additive is used as organogelator in an organic solvent, in order to obtain an organogel, or in a composition in an organic solvent medium, in particular in a coating composition from paints, varnishes, inks or gel coats, or in an adhesive composition or in a cosmetic composition or in a moulding composition or in a mastic or leaktightness agent or stripping agent composition.

16. Gel from organogel or hydrogel, in particular hydrogel, **characterized in that** it is the product resulting from the use as defined according to one of Claims 13 to 15 of an additive as defined according to one of Claims 1 to 5 or obtained by a process as defined according to Claim 6.

17. Final product resulting from the use in an aqueous medium, as hydrogelator and more particularly as rheology additive, of at least one additive as defined according to one of Claims 1 to 5 or obtained by a process as defined according to Claim 6, in particular selected from coating or adhesive or cosmetic or detergent or stripping agent or depolluting agent or treated fibre or treated textile.
